# EUROPÄISCHE PATENTSCHRIFT

(11) **EP 3 600 167 B1**
(45) Veröffentlichungstag und Bekanntmachung des Hinweises auf die Patenterteilung: **14.10.2020**
(21) Anmeldenummer: 18713589.2
(22) Anmeldetag: 19.03.2018
(51) Int. Cl.: A61F 2/78, A61F 2/80, D01F 6/06, B33Y 70/00

(54) **LINER FÜR EINE PROTHESE**
LINER FOR A PROSTHESIS
MANCHON POUR UNE PROTHÈSE

(30) Priorität: 30.03.2017 DE 102017106903
(43) Veröffentlichungstag der Anmeldung: 05.02.2020
(73) Patentinhaber: Ottobock SE & Co. KGaA, 37115 Duderstadt (DE)
(72) Erfinder: FINKE, Lars Benjamin, 37136 Landolfshausen (DE)
(74) Vertreter: Gramm, Lins & Partner Patent- und Rechtsanwälte PartGmbB
(86) Internationale Anmeldenummer: PCT/EP2018/056814
(87) Internationale Veröffentlichungsnummer: WO 2018/177783

(56) Entgegenhaltungen:
- EP-B1- 1 165 865
- DE-A1- 10 153 796
- DE-A1-102012 022 484
- US-A1- 2013 274 896

## Beschreibung

Die Erfindung betrifft einen Liner für eine Prothese, wobei der Liner aus einem Liner-Material hergestellt ist und eine proximale Öffnung zum Aufnehmen eines Amputationsstumpfes, ein distales Ende und eine Längsrichtung aufweist, die sich von der proximalen Öffnung zum distalen Ende erstreckt und ein auxetisches Material aufweist.

Derartige Liner sind aus dem Stand der Technik seit langem bekannt und werden über einen Amputationsstumpf gezogen, bevor dieser in einen Prothesenschaft eingeführt wird. Der Liner kann dabei unterschiedliche Aufgaben erfüllen.

Es ist bekannt, den Prothesenschaft am Amputationsstumpf zu halten, indem ein Volumen zwischen dem Prothesenschaft und dem Liner, der über den Amputationsstumpf gezogen ist, evakuiert wird. In diesem Fall wird die Prothese ausschließlich aufgrund des in dem Volumen herrschenden Unterdruckes am Amputationsstumpf gehalten. Dafür ist es selbstverständlich notwendig, dass das Volumen luftdicht abgeschlossen ist, wozu insbesondere der proximale Rand des Prothesenschaftes luftdicht am Liner anliegen muss. Nachteilig an dieser Art der Versorgung ist, dass das System komplex und die Handhabung aufwendig und schwierig ist.

Alternativ zu dieser Unterdrucklösung besteht daher die Möglichkeit am distalen Ende des Liners eine mechanische Befestigungseinrichtung, beispielsweise einen Pin oder eine ähnliche Einrichtung vorzusehen, mit der der Liner an einem entsprechend ausgebildeten Gegenstück im Inneren des Prothesenschaftes angeordnet werden kann. Ein Vakuum zwischen dem Prothesenschaft und dem Prothesenliner ist in diesem Fall nicht nötig oder wird allenfalls unterstützend eingesetzt.

Beim Gehen kommt es bei jedem Schritt in der Schwungphase zu einer in distaler Richtung wirkende Kraft, durch die der Prothesenschaft am Amputationsstumpf zieht. Es wird also eine entlang der Längsrichtung des Liners wirkende Kraft aufgebracht. Insbesondere bei Linern, die mechanisch am distalen Ende im Inneren des Prothesenschaftes befestigt sind, wird diese distal wirkende Zugkraft auf den Liner direkt übertragen, die eine Längung des Liners entlang seiner Längsrichtung hervorzurufen versucht. Ist der Liner beispielsweise aus Silikon oder einem anderen elastischen Material hergestellt, führt eine derartige Verlängerung in Längsrichtung zu einer Reduzierung des Umfanges und es kommt zum sogenannten "Melkeffekt", der unangenehm und zu vermeiden ist.

Zudem haben Amputationsstümpfe die Tendenz, im Laufe beispielsweise eines Tages, ihr Volumen zu verändern. Ein ausschließlich aus einem elastischen Material hergestellter Liner kann einer derartigen Volumenänderung folgen, indem beispielsweise bei einer Volumenabnahme die elastische Dehnung des Linermaterials abnimmt und so der Umfang des Liners dem schrumpfenden Umfang des Amputationsstumpfes folgt. Analog kann der elastische Liner sich weiter aufweiten, sofern das Volumen des Amputationsstumpfes sich im Laufe eines Tages vergrößert, sofern der den Liner umgebende starre Prothesenschaft dies erlaubt.

Aus dem Stand der Technik sind unterschiedliche Ansätze bekannt, auf derartige Volumenänderungen zu reagieren. Aus der DE 10 2012 017 324 A1 beispielsweise ist es bekannt, in das elastische Linermaterial unelastische Fasern derart einzulegen, dass eine Verlängerung des Liners entlang seiner Längsrichtung eine Verkürzung in Umfangsrichtung zur Folge hat und umgekehrt. Bei einer Volumenzunahme müsste sich folglich der Umfang vergrößern, was gleichzeitig eine Verkürzung des Liners in Längsrichtung zur Folge hätte. Dazu müsste der Liner an der Haut des Trägers entlang gleiten, was aufgrund der Hafteigenschaften nicht möglich ist. Auf diese Weise sollen Volumenänderungen möglichst unterbunden werden.

Die US 2013/0274896 A1 schlägt einen anderen Weg vor. Hier wird innerhalb des Liners ein dreidimensional auxetisches Material verwendet, das als auxetischer Schaum ausgebildet ist. Auxetische Materialien haben negative Poissonzahlen. Dies bedeutet, eine Verlängerung des Materials in einer Richtung führt anders als bei herkömmlichen Materialien nicht zu einer Verkürzung in einer jeweils anderen Richtung, sondern hat auch in diesen Richtungen eine Verlängerung zur Folge. Das dreidimensional auxetische Material aus dem Stand der Technik dehnt sich folglich aus, sofern das Volumen des Liners schrumpft. Dazu ist es notwendig, dass das Material sowohl mit dem Amputationsstumpf als auch mit der Innenseite des Prothesenschaftes so verbunden ist, dass es bei einer Volumenverringerung des Amputationsstumpfes nicht abgelöst wird. Dadurch kann es auf die Vergrößerung des Abstandes zwischen Amputationsstumpf und Prothesenschaft reagieren und den sich bildenden Raum aufgrund seiner dreidimensional auxetischen Eigenschaft ausfüllen. Dadurch soll ein sicherer Halt des Prothesenschaftes auch an einem sich verkleinernden Amputationsstumpf gewährleistet werden. Nachteilig ist jedoch, dass sich das dreidimensional auxetische Material auch dann ausdehnt, wenn eine Kraft in einer anderen Richtung wirkt, beispielsweise während der Schwungphase eines Gangzyklus. Auch während der Schwungphase hat das auxetische Material folglich die Tendenz sich auszudehnen und so den Melkeffekt zu verstärken und eine Volumenabnahme des Amputationsstumpfes zu forcieren.

Die US 2005/0101693 A1 beschreibt einen Liner, der in unterschiedlichen Bereichen unterschiedliche Elastizitäten aufweist und insbesondere mit einem PolymerGel getränkt oder beschichtet sein kann.

Der Erfindung liegt die Aufgabe zugrunde, einen Liner gemäß dem Oberbegriff des Anspruchs 1 so zu verbessern, dass die genannten Nachteile verhindert oder zumindest verringert werden.

Die Erfindung löst die gestellte Aufgabe durch einen Liner gemäß dem Oberbegriff des Anspruchs 1, der sich dadurch auszeichnet, dass das auxetische Material ein zweidimensional auxetisches Material ist, das derart angeordnet ist, dass eine Verlängerung des Liners in Längsrichtung zu einer Aufweitung eines Durchmessers des Liners führt, sodass dabei auf einen in dem Liner angeordneten Amputationsstumpf wirkende Zugkräfte auch in radialer Richtung wirken, wobei sich bei einem zwei-dimensional auxetischen Material bei einer Zugkraft in eine erste Richtung auch die Ausdehnung des Materials in einer zu der ersten Richtung senkrecht stehenden zweiten Richtung vergrößert, ohne dass die Ausdehnung in der dritten senkrecht stehenden Richtung nennenswert verändert wird.

Zweidimensional auxetische Materialen und Verfahren zu ihrer Herstellung werden beispielsweise in der EP 1 165 865 B1 beschrieben. Auxetische Materialien verfügen über eine negative Querdehung beziehungsweise eine negative Poissonzahl. Bei zweidimensional auxetischen Materialien vergrößert sich bei einer Zugkraft in eine erste Richtung die Ausdehung des Materials auch in einer zu der ersten Richtung beispielsweise senkrecht stehenden zweiten Richtung, ohne dass die Ausdehnung in der dritten senkrecht stehenden Richtung nennenswert verändert wird. Vorzugsweise findet in dieser dritten Richtung gar keine Veränderung statt.

Insbesondere während einer Schwungphase eines Gangzyklus kommt es, wie bereits dargelegt, zu in Längsrichtung wirkenden Zugkräften, die auf den Liner für die Prothese wirken. Aufgrund der zweidimensional auxetischen Eigenschaften kommt es dabei zu einer Aufweitung des Durchmessers des Liners, so dass die wirkenden Zugkräfte, die auf den Amputationsstumpf übertragen werden, nicht allein entlang der Längsrichtung, sondern insbesondere auch in radialer Richtung wirken. Dies hat einerseits eine möglichst homogene Verteilung der auftretenden Kräfte zur Folge und führt andererseits dazu, dass Volumenänderungen, insbesondere einer Volumenabnahme, aktiv entgegengewirkt wird. Es ist mit dem erfindungsgemäßen Liner nicht notwendig, einen sich bildenden Zwischenraum zwischen einem schrumpfenden Amputationsstumpf und dem Prothesenschaft durch ein sich ausdehnendes dreidimensional auxetisches Material zu füllen, da durch das verwendete zweidimensional auxetische Material dieser Volumenabnahme des Amputationsstumpfes entgegengewirkt wird. Eine Ausdehnung des zweidimensional auxetischen Materials in radialer Richtung bezüglich der Längsrichtung, also eine Vergrößerung der Dicke des Liners findet erfindungsgemäß nicht statt.

Vorteilhafterweise verfügt der Liner über ein Befestigungselement, vorzugsweise ein Formschlusselement, insbesondere einen Pin, mit dem er in einer Ausnehmung des Prothesenschaftes einführbar und so an diesem befestigbar ist. Natürlich kann zusätzlich oder alternativ auch wenigstens eine Kraftschlusselement, beispielsweise ein glatter Pin verwendet werden. Anstelle eines Pins kann beispielsweise auch ein Klettverschlusselement oder ein sonstiges Formschlusselement verwendet werden. Durch die erfindungsgemäße Ausgestaltung ist es möglich, die mechanische Verbindung des Liners im distalen Bereich mit dem Prothesenschaft zu verwenden, ohne dass es zu dem bekannten Melkeffekt kommt. Zumindest wird dieser Effekt stark vermindert. Damit ist es möglich, die nur im Bereich der Formschlusselemente auf den Liner wirkenden Zugkräfte, die während der Schwungphase eines Gangzyklus aufgebracht werden, als in radialer Richtung wirkende Kräfte auf den gesamten Liner und damit auf den Amputationsstumpf möglichst gleichmäßig zu verteilen. Der Melkeffekt tritt nicht ein. Damit kann diese möglichst homogene Kraftverteilung, die prinzipiell aus Unterdruckverbindungen bekannt ist, mit der sicheren und als vertrauenswürdiger eingestuften Verbindung des Liners im distalen Bereich mit dem Prothesenschaft kombiniert werden. Dazu ist keine pneumatische Abdichtung gegen die Umgebung nötig.

Vorzugsweise verfügt der Liner an einem distalen Ende über wenigstens ein Zugelement, beispielswese einen Gurt oder ein Gurtsystem, mit dem der Liner in einen Prothesenschaft eingezogen werden kann. Dazu wird das jeweilige Zugelement durch eine dafür am Prothesenschaft vorhandene Ausnehmung gezogen. Anschließend kann das Zugelement beispielsweise auf der Außenseite des Prothesenschaftes befestigt werden.

Vorzugsweise werden unterschiedliche Haltemechanismen kombiniert, wobei immer auch ein auxetisches Material verwendet wird. So kann beispielsweise im distalen Bereich eine Unterdruckversorgung verwendet werden. Dazu weist der Liner vorzugsweise wenigstens eine Dichtlippe auf, die ein distales Volumen zwischen dem Liner und dem Prothesenschaft luftdicht abdichtet. Im proximalen Bereich des Liners wird vorzugsweise das zweidimensional auxetische Material verwendet.

Vorteilhafterweise ist das auxetische Material in das Linermaterial eingebettet, vorzugsweise eingegossen, oder auf eine Außenseite des Linermaterials aufgebracht. Auf diese Weise lässt sich dieser Liner besonders einfach herstellen.

Vorzugsweise bildet das auxetische Material eine separate Schicht, die mit dem Linermaterial vorzugsweise stoffschlüssig und/oder formschlüssig verbunden ist.

Die Schicht kann beispielsweise in einem separaten Verfahrensschritt beispielsweise durch einen 3D-Druck mit einem 3D-Drucker hergestellt und anschließend mit dem Linermaterial verbunden werden. Dies kann auch nach dem Herstellen des Linermaterials, also insbesondere nach dem Aushärten, Abkühlen oder Vernetzen des Linermaterials geschehen.

Vorteilhafterweise ist das auxetische Material derart mit dem Verbindungselement verbunden, dass Zug- und/oder Druckkräfte übertragen werden können. Die beim Verwenden der Prothese auftretenden Zug- und/oder Druckkräfte können auf diese Weise über das Verbindungselement übertragen werden. Sie entfalten dann in dem auxetischen Material die bereits beschriebene Wirkung.

Vorzugsweise verfügt der Liner über eine rutschhemmende Beschichtung auf einer Innenseite, die im angelegten Zustand an der Haut des Trägers anliegt.

Vorzugsweise ist das auxetische Material durch ein Textil gebildet oder als Plattenwerkstoff ausgebildet. Die auxetische Eigenschaft kann vorzugsweise erreicht werden, indem beispielsweise nicht benötigte Elemente und/oder Teile und/oder Bereiche des plattenförmigen Werkstoffes entfernt werden, insbesondere durch Cutten oder Stanzen. Auch gegossene oder Laser-geschnittene auxetische Materialien können verwendet werden. Diese plattenförmigen auxetischen Materialien werden dann konfektioniert, so dass sie auf die jeweils benötigte Größe und Form gebracht und für den Liner verwendet werden können.

Vorteilhafterweise ist das auxetische Material durch einen 3D-Drucker hergestellt oder herstellbar. Das auxetische Material kann ein Kunststoff sein, der in bestimmte Strukturen, die zweidimensional auxetische Eigenschaften aufweisen, gebracht wird. Dies kann vorteilhafterweise durch einen 3D-Drucker entstehen. In diesem Fall lässt sich das zweidimensional auxetische Material leicht an die gewünschte Form, die beispielsweise durch die Größe und/oder Ausgestaltung des Liners bestimmt wird, anpassen. Das Material kann individuell hergestellt werden, so dass eine optimale Passform und eine optimale Kopplung zwischen Liner und Haut des Trägers bereitgestellt werden kann.

Vorzugsweise kann das auxetische Material auf das Linermaterial aufgedruckt werden.

Vorzugsweise ist das auxetische Material Teil eines Textils, vorzugsweise eines Gestrickes, besonders bevorzugt eines Rundgestrickes, das eine Außenschicht des Liners bildet. Dadurch wird die Herstellung des Liners weiter vereinfacht.

Ein Liner gemäß den hier beschriebenen Ausführungsformen kann beispielsweis in einem Spritzgussverfahren, insbesondere einem so genannten 2K-Verfahren hergestellt werden. So kann beispielsweise in einer Linerform, in der der Liner produziert wird, zunächst das auxetische Material beispielsweise in einem ersten Spritzgussschritt hergestellt werden. Anschließend wird das Linermaterial in die gleiche Linerform gegossen. Alternativ kann auch das Linermaterial, beispielsweise ein Elastomermaterial, vorproduziert werden. Auf dieses Linermaterial kann das auxetische Material aufgegossen oder aufgespritzt werden. Alternativ kann auch das auxetische Material vorproduziert und als Einlegeteil in die Linerform eingelegt werden, bevor das Linermaterial gegossen oder gespritzt wird.

In der Schwungphase eines Schrittes legt sich der Liner an den Schaft an und kann dafür sorgen, dass das Stumpfvolumen konstant bleibt. Dies kann auch zu einer besseren Durchblutung des Amputationsstumpfes führen.

Mit Hilfe der beiliegenden Zeichnungen wird nachfolgend ein Ausführungsbeispiel der vorliegenden Erfindung erläutert. Es zeigt:
- Figur 1 -: die schematische Wirkweise eines beispielhaften zweidimensional auxetischen Materials,
- Figur 2 -: die schematische Darstellung eines Liners gemäß einem ersten Ausführungsbeispiel der vorliegenden Erfindung,
- Figur 3 -: die Wirkweise eines Liners gemäß einem Ausführungsbeispiel der vorliegenden Erfindung,
- Figur 4 -: die schematische Darstellung eines weiteren auxetischen Materials und
- Figur 5 -: die Wirkweise eines Liners gemäß einem weiteren Ausführungsbeispiel

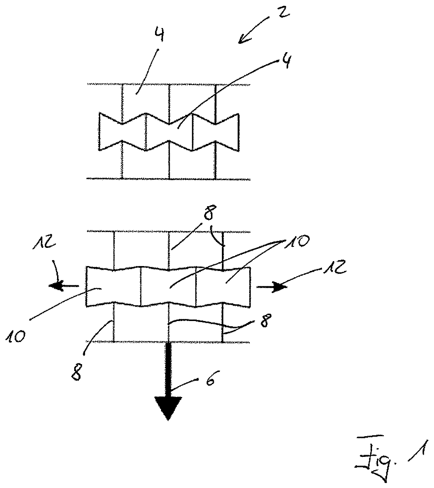

Figur 1 zeigt ein zweidimensional auxetisches Material 2. Im oberen Bereich ist es im entspannten unbelasteten Zustand dargestellt. Es weist eine spezielle Form mit unterschiedlichen Maschen oder Zellen 4 auf. Die einzelnen Begrenzungen dieser Zellen 4 können dabei aus einem herkömmlichen Kunststoff, der beispielsweise durch einen 3D-Drucker druckbar ist, bestehen. Durch die spezielle geometrische Form der verschiedenen Zellen 4 hat das auxetische Material 2 eine negative Poissonzahl. Dies wird im unteren Bereich der Figur 1 dargestellt. Es wird eine Zugkraft entlang des ersten Pfeiles 6 ausgeübt, die über die vertikal dargestellten Zellenwände 8 auf expandierbare Zellen 10 übertragen wird. Die jeweiligen Seitenwände werden, wie der Vergleich mit dem oberen Bereich von Figur 1 deutlich zeigt, auseinandergezogen, wodurch sich auch ihre Länge in Querrichtung, in Figur 1 also von links nach rechts, vergrößert. Dadurch wird das auxetische Material 2 nicht nur in Richtung entlang des ersten Pfeiles 6 größer und verlängert sich, sondern expandiert auch in einer senkrecht dazu liegenden Richtung, die durch die zweiten Pfeile 12 dargestellt ist. Eine Verdickung oder Ausdehnung in einer dritten, senkrecht auf die beiden ersten Richtungen stehenden Richtung, die in Figur 1 senkrecht zur Zeichenebene stünde, findet nicht statt.

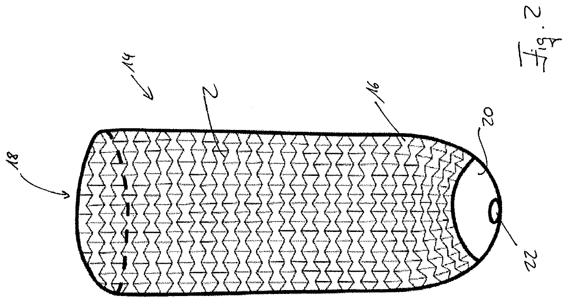

Figur 2 zeigt einen Liner 14, der aus einem Linermaterial 16 hergestellt ist und das zweidimensional auxetische Material 2 aufweist. Der Liner 14 verfügt über eine proximale Öffnung 18 zum Aufnehmen eines Amputationsstumpfes sowie ein distales Endes 20, das im gezeigten Ausführungsbeispiel ein Formschlusselement 22 aufweist. Im Bereich des distalen Endes 20 kann zudem eine Kappe oder Tasse angeordnet sein, durch die die über das Formschlusselement 22 übertragenen Zugkräfte im angelegten Zustand des Liners, wenn dieser mit einem nicht dargestellten Prothesenschaft verbunden ist, sicher übertragen werden können.

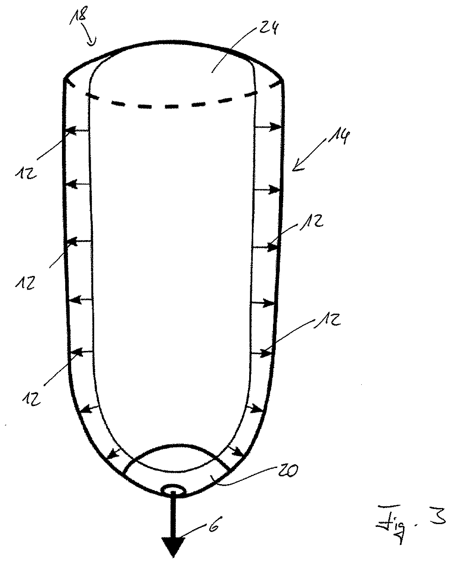

Figur 3 zeigt die schematische Darstellung des Liners 14, in dem sich ein Amputationsstumpf 24 befindet. Man erkennt die proximale Öffnung 18 und das distale Ende 20, auf das im gezeigten Ausführungsbeispiel eine Zugkraft entlang des ersten Pfeils 6 aufgebracht wird. Auf die Darstellung des auxetischen Materials 2 wurde aus Übersichtlichkeitsgründen verzichtet. Auch der Liner 14 im in Figur 3 gezeigten Ausführungsbeispiel weist jedoch dieses zweidimensional auxetische Material 2 auf.

Die auf das distale Ende 20 entlang des ersten Pfeils 6 aufgebrachte Zugkraft hat eine Verlängerung des Liners 14 entlang seiner Längsrichtung, die sich von der proximalen Öffnung 18 zum distalen Ende 20 erstreckt, zur Folge. Da das Material zweidimensional auxetisch ist, hat diese Verlängerung entlang der Längsrichtung auch eine Aufweitung des Liners und damit eine Vergrößerung des Durchmessers des Liners 14 zur Folge. Dies ist durch die zweiten Pfeile 12 dargestellt. Es wird folglich eine Zugkraft auf den Amputationsstumpf 24 übertragen, die im Idealfall auf die gesamte Fläche des Amputationsstumpfes 24 wirkt, die mit einer Innenseite des Liners 14 in Kontakt kommt. Dadurch wird die Zugkraft entlang der Richtung des ersten Pfeiles 6 einerseits homogen auf den Liner 14 und damit auch auf den Amputationsstumpf 24 verteilt und wirkt andererseits nicht mehr nur entlang der Längsrichtung, sondern sorgt für eine in radialer Richtung wirkende Kraft entlang der zweiten Pfeile 12. In jedem Schrittzyklus wird in der Schwungphase eine entsprechende Kraft aufgebracht, die folglich einer Volumenverkleinerung des Amputationsstumpfes 24 entgegenwirkt.

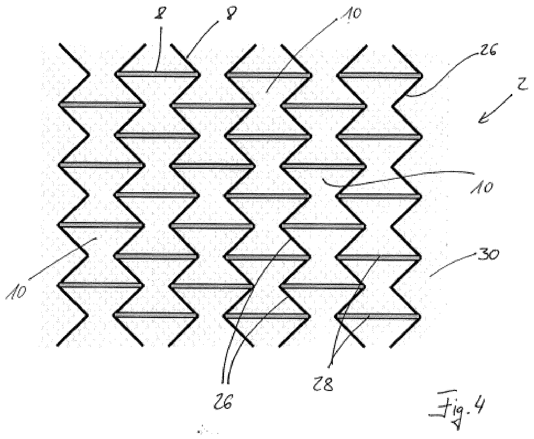

Figur 4 zeigt ein weiteres auxetisches Material 2, das die in Figur 1 gezeigte Struktur aufweist. Es verfügt über expandierbare Zellen 10, die durch Zellenwände 8 begrenzt sind.

Die Zellenwände 8 sind zickzack-förmig angeordnete, zugstabile Wände 26, zwischen denen druckstabile Verstärkungselemente 28 angeordnet sind, die die expandierbaren Zellen 10 begrenzen. Die zugstabilen Wände 26 und die druckstabilen Verstärkungselemente 28 sorgen für die auxetische Eigenschaft des auxetischen Materials 2. Sie sind in ein Gewebe 30 eingebettet, das bidirektional elastisch ist. Dabei können die zugstabilen Wände 26 und die druckstabilen Verstärkungselemente 28 auf das Material aufgebracht, beispielsweise aufgeklebt, beschichtet oder aufgedampft sein, oder in das Gewebe 30 integriert sein.

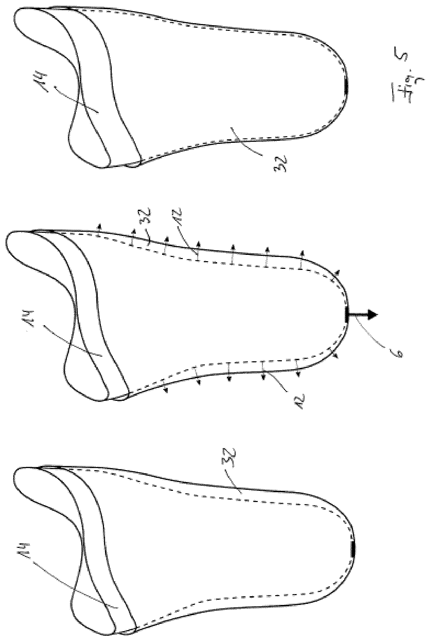

Figur 5 zeigt einen Liner 14 in einem Prothesenschaft 32. Durch eine gestrichelte Linie ist der Liner 14 dort dargestellt, wo er durch den Prothesenschaft 32 nicht zu sehen ist.

In der linken Darstellung der Figur 5 ist der unbelastete Zustand dargestellt. Im mittleren Teil hingegen wirkt eine Kraft entlang des ersten Pfeiles 6 auf den Liner 14. Diese Kraft kann beispielsweise in der Schwungphase eines Schrittes hervorgerufen werden. Durch die auxetischen Eigenschaften des Materials des Liners 14 dehnt sich der Liner entlang der Richtungen aus, die durch die zweiten Pfeile 12 dargestellt werden. Dadurch wird die in Figur 5 rechts dargestellte Situation erreicht, in der der Liner 14 und der sich darin befindende, nicht gezeigte Amputationsstumpf den Prothesenschaft 32 optimal ausfüllen.

### Bezugszeichenliste

- 2: auxetisches Material
- 4: Zellen
- 6: erster Pfeil
- 8: Zellenwände
- 10: expandierbare Zelle
- 12: zweiter Pfeil
- 14: Liner
- 16: Linermaterial
- 18: proximale Öffnung
- 20: distales Ende
- 22: Formschlusselement
- 24: Amputationsstumpf
- 26: zugstabile Wand
- 28: druckstabiles Verstärkungselement
- 30: Gewebe
- 32: Prothesenschaft

## Patentansprüche

1. Liner (14) für eine Prothese, wobei der Liner (14)
- aus einem Linermaterial (16) hergestellt ist und
- eine proximale Öffnung (18) zum Aufnehmen eines Amputationsstumpfes (24),
- ein distales Ende (20),
- eine Längsrichtung aufweist, die sich von der proximalen Öffnung (18) zum distalen Ende (20) erstreckt und
- ein auxetisches Material aufweist
**dadurch gekennzeichnet, dass** das auxetische Material ein zwei-dimensional auxetisches Material (2) ist, das derart angeordnet ist, dass eine Verlängerung des Liners (14) in Längsrichtung zu einer Aufweitung eines Durchmessers der Liners (14) führt, sodass dabei auf einen in dem Liner angeordneten Amputationsstumpf wirkende Zugkräfte auch in radialer Richtung wirken, wobei sich bei einem zwei-dimensional auxetischen Material bei einer Zugkraft in eine erste Richtung auch die Ausdehnung des Materials in einer zu der ersten Richtung senkrecht stehenden zweiten Richtung vergrößert, ohne dass die Ausdehnung in der dritten senkrecht stehenden Richtung nennenswert verändert wird.

2. Liner (14) nach Anspruch 1, **dadurch gekennzeichnet, dass** der Liner (14) ein Befestigungselement, vorzugsweise ein Formschlusselement (22), insbesondere einen Pin aufweist, mit dem er in eine Ausnehmung eines Prothesenschaftes einführbar und so an diesem befestigbar ist.

3. Liner (14) nach Anspruch 1 oder 2, **dadurch gekennzeichnet, dass** das auxetische Material (2) in das Linermaterial (16) eingebettet, insbesondere eingegossen, oder auf einer Außenseite des Linermaterials (6) aufgebracht ist.

4. Liner (14) nach Anspruch 1 oder 2, **dadurch gekennzeichnet, dass** das auxetische Material (2) eine separate Schicht bildet, die mit dem Linermaterial (16) vorzugsweise kraftschlüssig verbunden ist.

5. Liner (14) nach einem der Ansprüche 2 bis 4, **dadurch gekennzeichnet, dass** das auxetische Material (2) derart mit dem Verbindungselement verbunden ist, dass Zug- und/oder Druckkräfte übertragen werden können.

6. Liner (14) nach einem der vorstehenden Ansprüche, **dadurch gekennzeichnet, dass** der Liner (14) eine rutschhemmende Beschichtung auf einer Innenseite aufweist, die im angelegten Zustand an der Haut eines Trägers anliegt.

7. Liner (14) nach einem der vorstehenden Ansprüche, **dadurch gekennzeichnet, dass** das auxetische Material (2) durch einen 3D-Drucker herstellbar oder hergestellt ist.

8. Liner (14) nach Anspruch 7, **dadurch gekennzeichnet, dass** das auxetische Material (2) auf das Linermaterial (16) aufgedruckt ist.

9. Liner (14) nach einem der vorstehenden Ansprüche, **dadurch gekennzeichnet, dass** das auxetische Material (2) Teil eines Textils, insbesondere eines Gestrickes, besonders bevorzugt eines Rundgestrickes, ist, dass eine Außenschicht des Liners (14) bildet.

## Claims

1. A liner (14) for a prosthesis, wherein the liner (14)
- is made of a liner material (16) and
- comprises a proximal opening (18) for accommodating an amputation stump (24),
- a distal end (20),
- a longitudinal direction, which extends from the proximal opening (18) to the distal end (20) and
- comprises an auxetic material,
**characterized by the fact that** the auxetic material is a two-dimensional-auxetic material (2), which is arranged in such a way that an extension of the liner (14) in the longitudinal direction leads to an expansion of a diameter of the liner (14) such that tensile forces acting thereby on the amputation stump also act in a radial direction, wherein in a two-dimensional auxetic material, when a tensile force is acting the expansion of the material increases in a first direction as well as in a second direction that is perpendicular to the first direction, without a significant change in the expansion in the third perpendicular direction.

2. The liner (14) according to claim 1, **characterized by** the fact that the liner (14) has a fixing element, preferably a positive-locking element (22), in particular a pin, with which said liner can be introduced into a recess of the prosthetic socket and thus fixed to it.

3. The liner (14) according to claim 1 or 2, **characterized by** the fact that the auxetic material (2) is embedded, in particular moulded, in the liner material (16) or applied to an outer side of the liner material (6).

4. The liner (14) according to claim 1 or 2, **characterized by** the fact that the auxetic material (2) forms a separate layer, which is preferably connected to the liner material (16) via a force-locking connection.

5. The liner (14) according to one of the claims 2 to 4, **characterized by** the fact that the auxetic material (2) is connected to the connecting element such that tensile and/or compressive forces can be transferred.

6. The liner (14) according to one of the above claims, **characterized by** the fact that the liner (14) comprises an anti-slip coating on an inner side, said coating lying closely on the skin of the wearer when the liner is mounted.

7. The liner (14) according to one of the above claims, **characterized by** the fact that the auxetic material (2) can be or is produced by a 3D printer.

8. The liner (14) according to claim 7, **characterized by** the fact that the auxetic material (2) is imprinted on the liner material (16).

9. The liner (14) according to one of the above claims, **characterized by** the fact that the auxetic material (2) is part of a textile, especially a knitted fabric, especially preferably a circular knitted, which forms an outer layer of the liner (14).

## Revendications

1. Garniture (14) pour une prothèse, dans laquelle ladite garniture (14)
- est fabriquée à partir d'un matériau de garniture (16),
et comporte
- une ouverture proximale (18) pour recevoir un moignon d'amputation (24),
- une extrémité distale (20),
- une direction longitudinale qui s'étend depuis l'ouverture proximale (18) jusqu'à l'extrémité distale (20),
et comprend
- un matériau auxétique,
**caractérisée en ce que**
le matériau auxétique est un matériau auxétique (2) bidimensionnel qui est agencée de telle manière qu'un allongement de la garniture (14) en direction longitudinale mène à un élargissement d'un diamètre de la garniture, de sorte que les forces de traction agissant sur un moignon d'amputation agencé dans la garniture agissent alors également en direction radiale, et dans le cas d'un matériau auxétique bidimensionnel sous une force de traction dans une première direction l'extension du matériau augmente également dans une seconde direction perpendiculaire à la première direction, sans que l'allongement dans la troisième direction perpendiculaire soit notablement modifié.

2. Garniture (14) selon la revendication 1, **caractérisée en ce que** la garniture (14) comprend un élément de fixation, de préférence un élément à coopération de formes (22), en particulier une broche, avec lequel elle peut être introduite dans un évidement d'une emboîture de prothèse est être ainsi fixée sur celle-ci.

3. Garniture (14) selon la revendication 1 ou 2, **caractérisée en ce que** le matériau auxétique (2) est noyé dans le matériau de garniture (16), en particulier par coulée, ou bien est appliqué sur une face extérieure du matériau de garniture (16).

4. Garniture (14) selon la revendication 1 ou 2, **caractérisée en ce que** le matériau auxétique (2) forme une couche séparée qui est reliée au matériau de garniture (16) de préférence par coopération de forces.

5. Garniture (14) selon l'une des revendications 2 à 4, **caractérisée en ce que** le matériau auxétique (2) est relié à l'élément de liaison de telle manière que des forces de traction et/ou des forces de compression peuvent être transmises.

6. Garniture (14) selon l'une des revendications précédentes, **caractérisée en ce que** la garniture (14) comporte sur un côté intérieur un revêtement réduisant le glissement qui, dans l'état appliqué, s'applique contre la peau d'un porteur.

7. Garniture (14) selon l'une des revendications précédentes, **caractérisée en ce que** le matériau auxétique (2) est fabriqué ou peut être fabriqué au moyen d'une imprimante 3D.

8. Garniture (14) selon la revendication 7, **caractérisée en ce que** le matériau auxétique (2) est imprimé sur le matériau de garniture (16).

9. Garniture (14) selon l'une des revendications précédentes, **caractérisée en ce que** le matériau auxétique (2) fait partie d'un textile, en particulier d'un tricot, de manière particulièrement préférée un tricot circulaire, qui forme une couche extérieure de la garniture (14).
